# EUROPÄISCHE PATENTSCHRIFT

(11) **EP 0 396 702 B1**
(45) Veröffentlichungstag und Bekanntmachung des Hinweises auf die Patenterteilung: **22.09.1993**
(21) Anmeldenummer: 89912403.6
(22) Anmeldetag: 14.11.1989
(51) Int. Cl.: A61F 13/06

(54) **ELASTISCHE KNIEGELENKBANDAGE**
ELASTIC KNEE BANDAGE
GENOUILLERE ELASTIQUE

(30) Priorität: 14.11.1988 DE 3838576
(43) Veröffentlichungstag der Anmeldung: 14.11.1990
(73) Patentinhaber: Bauerfeind GmbH & Co., D-47880 Kempen (DE)
(72) Erfinder: HESS, Heinrich, D-6630 Saarlouis (DE); KRAUSE, Wolfgang, D-3500 Kassel (DE); BAUERFEIND, Hans, B., D-4152 Kempen 1 (DE)
(74) Vertreter: Bardehle, Heinz, Dipl.-Ing.
(86) Internationale Anmeldenummer: DE8900713
(87) Internationale Veröffentlichungsnummer: WO9005510

(56) Entgegenhaltungen:
- WO-A-85/04569
- DE-A- 3 416 231
- US-A- 4 116 236
- US-A- 4 445 505

## Beschreibung

Die Erfindung bezieht sich auf eine elastische Kniegelenkbandage in Schlauchform mit einer elastischen, die Kniescheibe in einer Aussparung umfassenden Profileinlage.

Eine derartige Kniegelenkbandage ist aus der DE-PS 34 12 772 bekannt. Diese Bandage enthält eine beidseitig mit Verlängerungslappen das Kniegelenk teilweise anfassende Profileinlage, wobei in die Verlängerungslappen Verstärkungsstücke eingebaut sind. Mit diesen Verstärkungsstücken soll auf das Kniegelenk von beiden Seiten ein radial wirkender Druck ausgeübt werden. Bei einer weiteren in der Druckschrift beschriebenen Ausführungsform ist die Profileinlage einseitig mit einer wulstförmigen Erhöhung in der Nähe der Aussparung für die Kniescheibe versehen, die ebenfalls an ihrer Stelle den Druck auf das Knie erhöht. Wie in der zugehörigen Beschreibung ausgeführt ist, soll hierdurch die Kniescheibe sowohl nach innen als auch in Richtung auf die Außenseite des Knies verschoben werden können. Ausgenutzt wird dabei also eine statische Wirkung des durch die Erhöhung ausgeübten Druckes auf das Knie.

Es ist weiterhin in der US-PS 44 45 505 eine elastische Kniegelenkbandage beschrieben, die mit einer Aussparung für die Kniescheibe versehen ist und in die Polster eingebaut sind, durch deren Lage eine seitliche Verschiebung der Kniescheibe verhindert werden soll. Um die Wirkung dieser Polster zu intensivieren, ist die Kniegelenkbandage mit elastischen Bändern versehen, die mittels Klettverschlüssen um die Bandage gespannt werden können, wodurch die Lage der Polster gesichert werden soll. Es handelt sich bei dieser Kniegelenkbandage also ebenfalls um eine durch statisch wirkenden Druck ausgeübte Einwirkung auf die Kniescheibenposition.

Eine Kniegelenkbandage mit der Tendenz der medialen Verschiebung der Kniescheibe ist aus dem DE-U-81 15 670.7 bekannt geworden. Diese Bandage enthält eine C-förmige Profileinlage (Patellapelotte), deren offene Seite medial (beineinwärtig) angeordnet ist. Die Kniegelenkbandage, in die die Profileinlage entweder eingewebt oder eingenäht ist, besteht aus zwei Hüllenhälften, von denen die mediale Hüllenhälfte in strafferer Webart als die beinauswärtigen (laterale) Hüllenhälfte ausgebildet ist. Dieser Kniegelenkbandage liegt die Aufgabe zugrunde, eine Entlastung von verbrauchten retropatellaren Knorpelflächen und zugleich eine Umbelastung auf unverbrauchte Knorpelflächen zu bewirken. Wegen der strafferen Webart der lateralen Hüllenhälfte soll die Profileinlage ständig elastisch gegen die laterale Seite der Kniescheibe gezogen werden (siehe Beschreibungsseite 5 letzter Satz), insbesondere auch Bewegungen des Kniegelenks. Daß dieser Effekt bei der nur C-förmig ausgebildeten Profileinlage aufgrund der unterschiedlichen Straffheit der Webart der beiden Hüllenhälften tatsächlich eintritt, ist nicht ohne weiteres ersichtlich, denn bei angelegter Kniegelenkbandage muß sich diese mit ihren beiden Hüllenhälften an die jeweilige Form des Kniegelenks anpassen, wobei die Längsdehnung der schwächer gewebten Hüllenhälften von der Dehnung der stärker gewebten Hüllenhälfte im wesentlichen bestimmt wird. Daß es bei der Gestaltung dieser bekannten Kniegelenkbandage im wesentlichen auf einen ständigen Druck gegen die laterale Seite der Kniescheibe ankommt, geht auch aus einem weiteren in der Druckschrift behandelten Ausführungsbeispiel hervor, wonach an die beiden Enden der C-förmigen Profileinlage ein die Kniegelenkbandage über ihre rückwärtige Seite umfassendes Spannband ansetzt, das vom Benutzer auf eine gewünschte ständige Spannung eingestellt werden kann.

Der Erfindung liegt die Aufgabe zugrunde, auf eine Kniescheibe, die aus ihrer Idealposition entweder krankhaft oder als sehr häufige Normvariante wadenbeinseitig verschoben ist, beim Beugen des Kniegelenks dynamisch einzuwirken und dabei die Kniescheibenlage zu korrigieren. Erfindungsgemäß geschieht dies dadurch, daß in die Profileinlage in festem Verbund mit dieser ein biegsames, nicht dehnbares Spannglied eingelagert ist, das die Bereiche der Profileinlage benachbart zu den Kniescheibenpolen und wadenbeinseitig im Bogen um die Kniescheibe derart verbindet, daß bei Vergrößerung der Entfernung dieser Bereiche beim Beugen des Kniegelenks der Abstand des Bogens von der Verbindungslinie der Kniescheibenpole und verringert wird und der betreffende Rand der Aiissparung der Profileinlage auf die benachbarte Seite der Kniescheibe diese medial verschiebend und zentrierend drückt.

Beim Beugen des Kniegelenks wird diese Kniegelenkbandage an ihrer Vorderseite über die Kniescheibe beträchtlich gedehnt. Diese Dehnung überträgt sich auf die Profileinlage, die ein integraler Bestandteil der Kniegelenkbandage ist, so daß sich die Bereiche der Profileinlage, die den Kniescheibenpolen benachbart sind, voneinander gegenüber der Strecklage des Kniegelenks entfernen. Aufgrund dieser Vergrößerung der Entfernung wird auf die Enden des Spanngliedes gewissermaßen ein Zug ausgeübt, so daß dieses in seinem mittleren Bereich an die betreffende Seite der Kniescheibe herangezogen wird. Hierbei übt der das Spannglied enthaltene Rand der Aiissparung in der Profileinlage einen seitlichen Druck auf die Kniescheibe aus, die somit zunehmend mit der Beugung des Knies an die Kniescheibe herangezogen wird. Es ergibt sich damit ein dynamischer Vorgang, indem der sonst möglichen Verschiebung der Kniescheibe ohne die erfindungsgemäße Bandage in dem gleichen Umfang entgegengewirkt wird, wie die Tendenz zu der Fehlverschiebung der Kniescheibe besteht. Je mehr nämlich das Kniegelenk bis zu etwa 90° gebeugt wird, desto mehr besteht die Tendenz zur Fehlverschiebung, der aufgrund entsprechend zunehmenden seitlichen Drucks auf die Kniescheibe durch das Spannglied in vollem Umfang entgegengewirkt wird.

Da funktionell-anatomisch eine Seitverschiebung der Kniescheibe in der Funktion bei angespannter Oberschenkelstreckmuskulatur nach innen praktisch nie vorkommt, sondern nur eine solche nach außen, ist demnach auch nur eine Korrektur sinnvoll, die ein Abgleiten der Kniescheibe nach außen angeht und eine mittlere Positionierung der Kniescheibe bewirkt. Der von dem Spannglied ausgeübte seitliche Druck, der über eine Weichteilschicht an der Kniescheibe ansetzt, wird bei Benutzung der erfindungsgemäßen Bandage, also in der Bewegung, in keinem Fall eine derartige kraft aufbringen, durch die die Position der Kniescheibe über die Idealstellung hinaus nach innen verschoben wird, da hierzu die Lage des Spanngliedes und die Dehnung der Profileinlage beim Beugen des Knies nicht ausreicht.

Vorteilhaft wird das Spannglied als nicht dehnbarer, biegbarer Strang, direkt neben dem kniescheibenseitigen Rand der Profileinlage, die Kniescheibe mindestens vom oberen bis zum unteren Kniescheibenpol umfassend, in die Profileinlage in festem Verbund mit dieser eingelagert. Aufgrund dieser Lage des Stranges übt dieser in seiner Funktion einen direkten Einfluß auf die Kniescheibe aus.

Um dem Strang den festen Verbund mit der Profileinlage zu geben, kann man die Enden des Stranges zu Ankern im Material der Profileinlage formen, die auf der dem Bogen des Stranges abgewandten Seite der Verbindungslinie zwischen den Kniescheibenpolen liegen. Diese Anker kann man vorteilhaft als Schlaufen ausbilden, es ist aber auch möglich, als Anker Verdickungen des Stranges vorzusehen.

Eine weitere Möglichkeit der Schaffung des festen Verbundes des Stranges mit der Profileinlage besteht darin, den Strang über seine Länge mit Ausnehmungen zu versehen, die von dem Material der Profileinlage ausgefüllt sind. In diesem Falle wird durch das Eindringen des Materials der Profileinlage in die Ausnehmungen, beispielsweise Löcher in einem nicht dehnbaren Kunststoffband, dafür gesorgt, daß beim Dehnen der Profileinlage über das gebeugte Knie jede Stelle längs des Spanngliedes fest mit dem umgebenen Material der Profileinlage verbunden bleibt.

In den Figuren sind Ausführungsbeispiele der Erfindung dargestellt. Es zeigen:
- Figur 1: einen das Kniegelenk umfassenden Abschnitt eines menschlichen Beines in gestreckter Lage mit über das Kniegelenk gezogener Kniegelenkbandage, und zwar in Draufsicht auf die Kniescheibe,
- Figur 2: das Bein von der Seite gesehen in gebeugter Lage mit übergezogener Kniegelenkbandage,
- Figur 3: eine flache Abwicklung der in die Kniegelenkbandage integrierten Profileinlage, bezogen auf die Strecklage des Knies,
- Figur 4: die Abwicklung der Profileinlage, bezogen auf das gebeugte Knie,
- Figur 5: die Abwicklung der Profileinlage mit Verdickungen als Anker des Stranges.

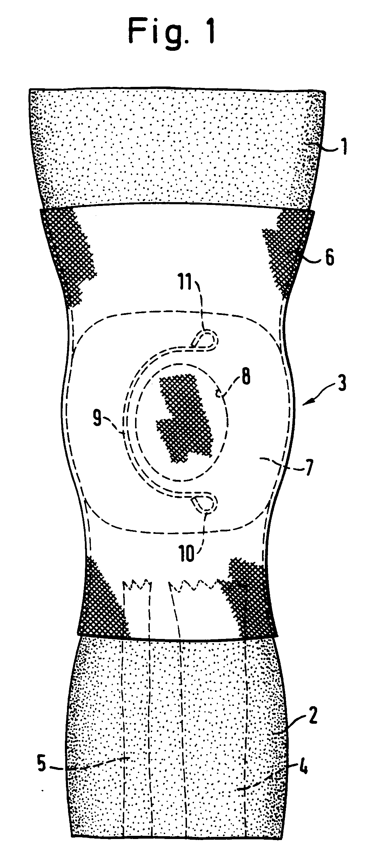

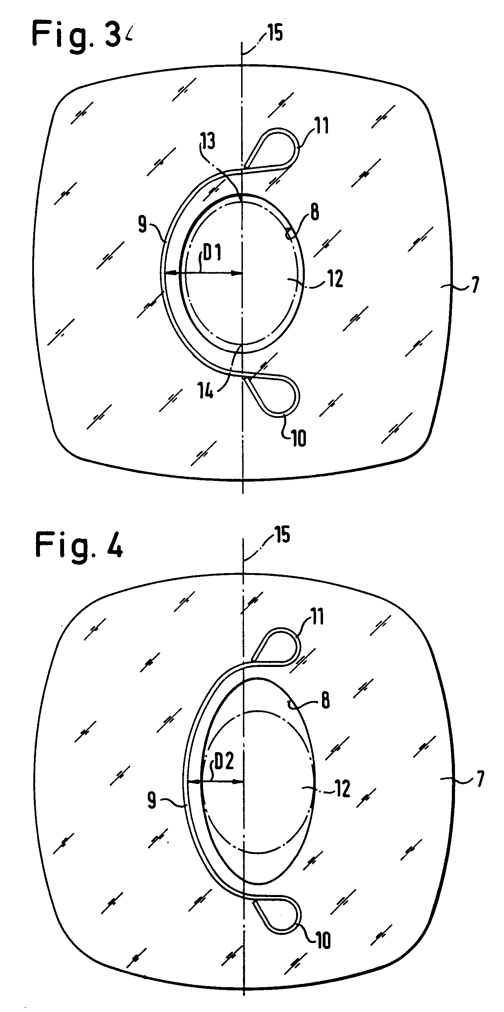

Figur 1 zeigt den Oberschenkel 1 und den Unterschenkel 2 sowie das Kniegelenk 3 eines menschlichen Beines, wobei im Unterschenkel 2 das Schienbein 4 und das Wadenbein 5 eingezeichnet sind. Über das Kniegelenk 3 ist die aus einem elastischen Textil bestehende Kniegelenkbandage 6 gezogen, die sich eng um das Kniegelenk 3 und die betreffenden Stellen des Oberschenkels 1 und des Unterschenkels 2 legt. In die Kniegelenkbandage 6 ist die anhand der Figuren 3 und 4 mehr im einzelnen beschriebene Profileinlage 7 eingelagert, die ein aus elastischem Material bestehendes Polster bildet. Als Material kann hierfür z. B. in bekannter Weise Silikonkautschuk verwendet werden. Die Profileinlage 7 ist im Bereich der hier nicht eingezeichneten Kniescheibe mit der Aussparung 8 versehen, die die Kniescheibe eng umfaßt. In die Profileinlage 7 ist der als Spannglied wirkende Strang 9 eingelagert, der z. B. als Drahtseil ausgebildet sein kann. Der Strang ist somit biegbar, aber nicht dehnbar, wodurch er in der Lage ist, Verformungen der Profileinlage 7 zu folgen, ohne sich dabei zu verlängern. An seinen Enden ist der Strang 9 mit den Schlaufen 10 und 11 versehen, die dem Strang 9 in der Profileinlage 7 eine sichere Verankerung geben, worauf im Zusammenhang mit den Figuren 3 und 4 näher eingegangen wird.

Die Profileinlage 7 wird zweckmäßig bei der Herstellung der Kniegelenkbandage 6 in diese eingearbeitet, es ist aber auch möglich, die Profileinlage auf die Bandage aufzukleben oder aufzuschweißen. In jedem Falle handelt es sich bei der Profileinlage 7 um einen integralen Bestandteil der Kniegelenkbandage 6 in dem Sinne, daß beim Beugen des Knies 3 sowohl die Kniegelenkbandage 6 als auch die Profileinlage 7 diese Bewegung mitmachen, ohne daß es dabei zwischen der Kniegelenkbandage 6 und der Profileinlage 7 zu einer Verschiebung kommen kann.

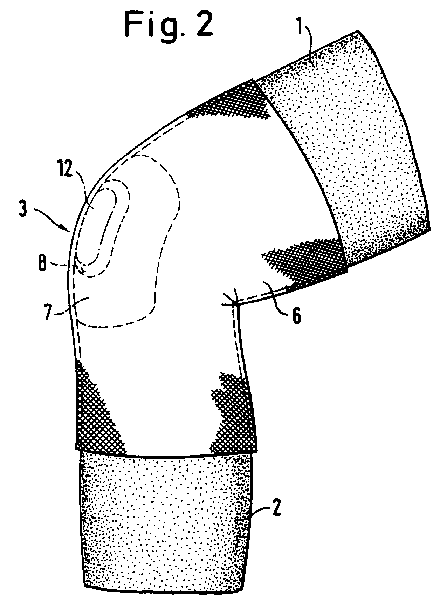

Figur 2 zeigt die gleiche Kniegelenkbandage 6 mit der darin enthaltenen Profileinlage 7 bei gebeugtem Knie 3. In der Figur 2 ist im Bereich der Aussparung 8 der Profileinlage 7 die Kniescheibe 12 in strichpunktierter Linienführung angedeutet.

Figuren 3 und 4 zeigen die Profileinlage 7 in flacher Abwicklung, und zwar gemäß Figur 3 bezogen auf das gestreckte Knie (siehe Figur 1) und Figur 4 bezogen auf das gebeugte Knie (siehe Figur 2). Darüber hinaus ist in den Figuren 3 und 4 noch in strichpunktierter Linienführung die Kniescheibe 12 eingezeichnet.

Die Profileinlage 7 gemäß Figur 3 enthält den Strang 9 mit den beiden als Anker dienenden Schlaufen 10 und 11. In Figur 3 ist die über die Pole 13 und 14 der Kniescheibe 12 verlaufende Mittellinie 15 eingezeichnet, die von dem Bogen des Stranges 9 den Abstand D1 einhält. Dieser Bogen umschlingt innerhalb des Materials der Profileinlage 7 die Kniescheibe 12 auf der Seite des Wadenbeins 5 (in Fig. 1) und verläuft dann über die Mittellinie 15 hinaus, wo der Strang 9 dann, wie gesagt, die Schlaufen 10 und 11 bildet. Die in der Figur 3 dargestellte Lage von Kniescheibe 12 und Profileinlage 7 stellt die für den betreffenden Patienten bestehende Normallage seiner Kniescheibe 12 dar - als Normvariante aber ohnehin schon leicht außenseitig der Idealposition gelegen - zusammen mit der die Kniescheibe 12 umgebenden Profileinlage 7.

Wenn nun das von der erfindungsgemäßen Kniegelenkbandage 6 umspannte Kniegelenk 3 gebeugt wird (siehe Figur 2), so ergibt sich für die Kniegelenkbandage 6 und die in ihr verankerte Profileinlage 7 auf der Außenseite des Kniegelenks 3 eine Dehnung, die dazu führt, daß die Aussparung 8 sich in Richtung der Mittellinie 15 streckt, wobei sie etwa eine ovalartige Form annimmt. Dies ist in Figur 4 dargestellt, wobei allerdings die Profileinlage 7 in der flachen Abwicklung gezeichnet ist. Da nun der Strang 9 aus nicht dehnbarem Material besteht und er in dem Material der Profileinlage 7 fest verankert ist, werden die Bereiche des Stranges 9 vor den Schlaufen 10 und 11 in Richtung der Mittellinie 15 auseinandergezogen, was wegen der Nichtdehnbarkeit des Stranges 9 zur Folge hat, daß dieser in seinem Bogenbereich an die Mittellinie 15 zu der heranrückt. Es verringert sich damit der Abstand des Bogenbereichs des Stranges 9 zur Mittellinie 15 auf die Länge D2. Dabei bleibt die Profileinlage 7 zusammen mit der Kniegelenkbandage 6 wegen der festen Umspannung des Kniegelenks 3 im Prinzip in ihrer Lage erhalten, so daß sich die Verringerung des Abstandes zwischen Bogenbereich des Stranges 9 und der Mittellinie 15 von der Länge D1 zu der Länge D2 derart auswirkt, daß der Rand 19 der Aussparung 8 von der Seite her gegen den betreffenden Rand der Kniescheibe 12 drückt und diese zur Beininnenseite hin verschiebt. Der Grad dieser Verschiebung ist abhängig vom Winkel der Beugung des Kniesgelenks 3.

Ausgehend von der in Figur 3 dargestellten Lage der Kniescheibe 12, die einer Fehllage entspricht, ergibt sich somit aufgrund der in der Figur 4 dargestellten seitlichen Verschiebung der Kniescheibe 12 eine Korrektur in die Idealposition, wobei es sich um eine Verschiebung um wenige Millimeter handelt, was die Figur 4 insofern zeigt, als sie etwa die Verhältnisse im Maßstab 1 : 1 zeigt.

Diese Lageveränderung der Kniescheibe 12 um nur wenige Millimeter erbringt medizinisch eine demgegenüber wesentliche Verbesserung der Druckverhältnisse im Kniescheiben-Oberschenkelgelenk, da nunmehr die Kniescheibe mit ihrer Innenfläche kongruent-ideal der Oberschenkelgelenkfläche in der Bewegung gegenübersteht.

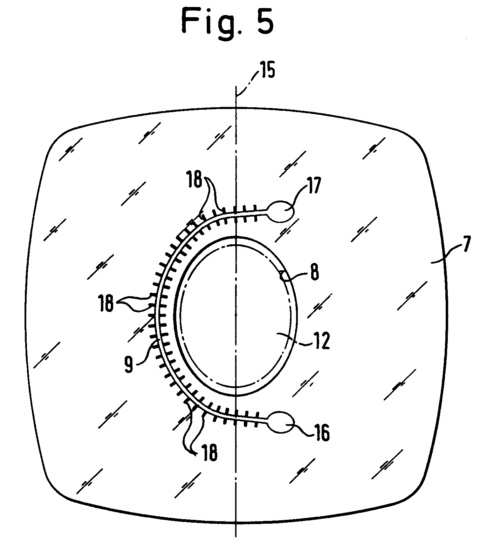

In Figur 5 ist eine Variante der Methode der Verankerung des Stranges 9 in der Profileinlage 7 dargestellt. Es handelt sich dabei um die beiden Verdickungen 16 und 17 an den Enden des Stranges 9, der hier aus einem biegsamen, nicht dehnbaren Kunststoffstrang besteht. Außerdem ist dargestellt, daß der Strang 9 über seine Länge mit einer Vielzahl von Abspreizungen 18 versehen ist, mit denen der Strang 9 in das Material der Profileinlage 7 eindringt und somit eine zusätzliche, möglicherweise auch für sich ausreichende Verankerung sorgt. Es sei noch darauf hingewiesen, daß die Verankerung des Stranges 9 über seine Länge z. B. auch dadurch herbeigeführt werden kann, daß der Strang mit Ausnehmungen, z.B. in Form von Löchern, versehen wird, die dann vom Material der Profileinlage 7 ausgefüllt werden. In diesem Falle verwendet man als Strang zweckmäßig ein leiterartig gelochtes Kunststoffband.

## Patentansprüche

1. Elastische Kniegelenkbandage (6) in Schlauchform mit einer elastischen, die Kniescheibe (12) in einer Aussparung (8) umfassenden Profileinlage (7), **dadurch gekennzeichnet,** daß in die Profileinlage (7) in festem Verbund mit dieser ein biegsames, nicht dehnbares Spannglied (9) eingelagert ist, das die Bereiche der Profileinlage (7) benachbart zu den Kniescheibenpolen (13,14) wadenbeinseitig (5) im Bogen um die Kniescheibe (12) derart verbindet, daß bei Vergrößerung der Entfernung dieser Bereiche beim Beugen des Kniegelenks (3) der Abstand des Bogens von der Verbindungslinie der Kniescheibenpole (13,14) verringert wird und der betreffende Rand der Aussparung (8) der Profileinlage (7) auf die benachbarte Seite der Kniescheibe (12) diese medial verschiebend und zentrierend drückt.

2. Kniegelenkbandage nach Anspruch 1, **dadurch gekennzeichnet,** daß das Spannglied als nicht dehnbarer, biegbarer Strang (9) ausgebildet ist, der direkt neben dem kniescheibenseitigen Rand der Profileinlage (7), die Kniescheibe (12) mindestens vom oberen bis zum unteren Kniescheibenpol (13,14) umfassend, in diese eingelagert ist.

3. Kniegelenkbandage nach Anspruch 2, dadurch gekennzeichnet, daß die Enden des Stranges (9) zu Ankern (10,11;16,17) im Material der Profileinlage (7) geformt sind, die auf der dem Bogen des Stranges (9) abgewandten Seite der Verbindungslinie zwischen den Kniescheibenpolen (13,14) liegen.

4. Kniegelenkbandage nach Anspruch 3, dadurch gekennzeichnet, daß die Anker als Schlaufen (10,11) ausgebildet sind.

5. Kniescheibengelenkbandage nach Anspruch 3, dadurch gekennzeichnet, daß die Anker als Verdickungen (16,17) des Stranges (9) ausgebildet sind.

6. Kniegelenkbandage nach einem der Ansprüche 2 - 5, dadurch gekennzeichnet, daß der Strang (9) über seine Länge mit Ausnehmungen, z.B. Löchern, versehen ist, die von dem Material der Profileinlage ausgefüllt sind.

## Claims

1. Elastic knee bandage (6) in tubular form with an elastic profiled insert (7) surrounding the knee-cap (12) in a recess (8), characterised in that a flexible, inextensible clamping member (9) is embedded into the profiled insert (7) so that it is firmly bonded thereto and connects the regions of the profiled insert (7) adjacent to the poles of the knee-cap (13, 14) and at the fibula side (5) in an arc around the knee-cap (12) in such a manner that as the distance between these regions increases during flexion of the knee joint (3), the distance between the arc and the line joining the poles of the knee-cap (13, 14) decreases and the corresponding edge of the recess (8) in the profiled insert (7) presses against the adjacent side of the knee-cap (12), displacing it medially and centering it.

2. Knee bandage according to claim 1, characterised in that the clamping member is in the form of an inextensible, flexible strand (9) which is embedded into the profiled insert (7) directly alongside the knee-cap edge thereof, surrounding the knee-cap (12) at least from the upper to the lower pole of the knee-cap (13, 14).

3. Knee bandage according to claim 2, characterised in that the ends of the strand (9) can be shaped into anchors (10, 11; 16, 17) in the material of the profiled insert (7), these being situated on the side of the line joining the poles of the knee-cap (13, 14) remote from the arc of the strand (9).

4. Knee bandage according to claim 3, characterised in that the anchors are in the form of loops (10, 11).

5. Knee bandage according to claim 3, characterised in that the anchors are in the form of thickened portions (16, 17) on the strand (9).

6. Knee bandage according to one of claims 2 - 5, characterised in that the strand (9) is provided over its length with recesses, e.g. holes, which are filled with the material of the profiled insert.

## Revendications

1. Bandage élastique (6) pour le genou, de forme tubulaire, avec une pièce d'insertion profilée (7) élastique, entourant la rotule (12) par un évidement (8), **caractérisé** en ce qu'un organe de serrage (9), flexible mais non extensible, est incorporé dans la pièce d'insertion profilée (7) en liaison fixe avec cette dernière, organe qui, du côté du péroné (5), relie en arc autour de la rotule (12) les régions de la pièce d'insertion profilée (7) voisines des pôles (13, 14) de la rotule de telle sorte que, tandis que la distance entre ces régions augmente lors de la flexion du genou (3), la distance entre l'arc et la droite de jonction des pôles (13, 14) de la rotule diminue et le bord correspondant de l'évidement (8) de la pièce d'insertion profilée (7) exerce une pression sur le côté voisin de la rotule (12), de sorte qu'il déplace médialement et centre cette dernière.

2. Bandage pour le genou selon la revendication 1, **caractérisé** en ce que l'organe de serrage est réalisé sous la forme d'un cordon (9), flexible mais non extensible, qui est incorporé dans la pièce d'insertion profilée (7) au voisinage immédiat du bord côté rotule de cette pièce, en entourant la rotule (12) au moins depuis le pôle supérieur (13) jusqu'au pôle inférieur (14) de la rotule.

3. Bandage pour le genou selon la revendication 2, **caractérisé** en ce que les extrémités du cordon (9) sont formées en éléments d'ancrage (10, 11 ; 16, 17) dans le matériau de la pièce d'insertion profilée (7), éléments qui se trouvent sur le côté de la droite de jonction entre les pôles (13, 14) de la rotule qui est opposé à l'arc du cordon (9).

4. Bandage pour le genou selon la revendication 3, **caractérisé** en ce que les éléments d'ancrage sont réalisés sous la forme de boucles (10, 11).

5. Bandage pour le genou selon la revendication 3, **caractérisé** en ce que les éléments d'ancrage sont réalisés sous la forme d'épaississements (16, 17) du cordon (9).

6. Bandage pour le genou selon l'une quelconque des revendications 2 à 5, **caractérisé** en ce que le cordon (9) est pourvu sur sa longueur d'évidements, par exemple de trous, qui sont remplis par le matériau de la pièce d'insertion profilée.
